# EUROPEAN PATENT SPECIFICATION

(11) **EP 1 805 510 B1**
(45) Date of publication and mention of the grant of the patent: **22.02.2012**
(21) Application number: 05777835.9
(22) Date of filing: 08.09.2005
(51) Int. Cl.: G01N 33/48, G01N 33/487, G01N 33/49, G01N 33/68, A61K 31/00, A61K 31/337, A61K 31/437, A61K 31/4745, A61K 39/00, A61K 39/395, A61P 25/00, A61P 25/16, A61P 37/00, A61P 37/06

(54) **THERAPEUTIC STRATEGY FOR TREATING AUTOIMMUNE AND DEGENERATIVE DISEASES**
THERAPIESTRATEGIE ZUR BEHANDLUNG VON AUTOIMMUNKRANKHEITEN UND DEGENERATIVEN ERKRANKUNGEN
STRATÉGIE THÉRAPEUTIQUE DESTINÉE AU TRAITEMENT DE MALADIES AUTO-IMMUNES ET DÉGÉNÉRATIVES

(30) Priority: 08.09.2004 AU 2004905118
(43) Date of publication of application: 11.07.2007
(73) Proprietor: Immunaid Pty Ltd, Fitzroy, Victoria 3065 (AU)
(72) Inventor: ASHDOWN, Maria, Luisa, Thornbury, Victoria 3071 (AU); ASHDOWN, Martin, Leonard, Thornbury, Victoria 3071 (AU)
(74) Representative: Soames, Candida Jane
(86) International application number: PCT/AU2005/001364
(87) International publication number: WO 2006/026821

(56) References cited:
- WO-A-02/45735
- WO-A-2005/040816
- WO-A2-02/45735
- GEORGE S K ET AL: "Immunokinetics of autoreactive CD4 T cells in blood: a reporter for the ''hit-and-run'' autoimmune attack on pancreas and diabetes progression" JOURNAL OF AUTOIMMUNITY, LONDON, GB, vol. 23, no. 2, 1 September 2004 (2004-09-01), pages 151-160, XP004590159 ISSN: 0896-8411
- HOLCOMBE HILDA ET AL: "The immunosuppressive agent 15-deoxyspergualin functions by inhibiting cell cycle progression and cytokine production following naive T cell activation." JOURNAL OF IMMUNOLOGY (BALTIMORE, MD. : 1950) 1 NOV 2002, vol. 169, no. 9, 1 November 2002 (2002-11-01), pages 4982-4989, XP002491621 ISSN: 0022-1767
- KEIMOWITZ R M: "DEMENTIA IMPROVEMENT WITH CYTOTOXIC CHEMOTHERAPY A CASE OF ALZHEIMER DISEASE AND MULTIPLE MYELOMA" ARCHIVES OF NEUROLOGY, AMERICAN MEDICAL ASSOCIATION, CHICAGO, IL, US, vol. 54, no. 4, 1 April 1997 (1997-04-01), pages 485-488, XP008024300 ISSN: 0003-9942
- RACKE M K ET AL: "INTRAVENOUS ANTIGEN ADMINISTRATION AS A THERAPY FOR AUTOIMMUNE DEMYELINATING DISEASE" ANNALS OF NEUROLOGY, BOSTON, US, vol. 39, no. 1, 1 January 1996 (1996-01-01), pages 46-56, XP008021103 ISSN: 0364-5134
- ELLIOTT M J ET AL: "SUPPRESSION OF FEVER AND THE ACUTE-PHASE RESPONSE IN A PATIENT WITH JUVENILE CHRONIC ARTHRITIS TREATED WITH MONOCLONAL ANTIBODY TO TUMOUR NECROSIS FACTOR-ALPHA (CA2)" BRITISH JOURNAL OF RHEUMATOLOGY, BAILLIERE TINDALL, LONDON, GB, vol. 36, 1 January 1997 (1997-01-01), pages 589-593, XP002953580 ISSN: 0263-7103
- HUGHES P J ET AL: "Dual labelling of circulating CD8 cells in patients with multiple sclerosis" JOURNAL OF NEUROLOGY, NEUROSURGERY, AND PSYCHIATRY JAN 1989,, vol. 52, no. 1, 1 January 1989 (1989-01-01), pages 118-121, XP002489211
- BON G G ET AL: "Fluctuations in CA 125 and CA 15-3 serum concentrations during spontaneous ovulatory cycles" HUMAN REPRODUCTION (OXFORD, ENGLAND) FEB 1999,, vol. 14, no. 2, 1 February 1999 (1999-02-01), pages 566-570, XP002489212
- VON HERRATH MATTHIAS G ET AL: "Antigen-induced regulatory T cells in autoimmunity." NATURE REVIEWS. IMMUNOLOGY MAR 2003, vol. 3, no. 3, March 2003 (2003-03), pages 223-232, XP002491622 ISSN: 1474-1733
- GEORGE S K ET AL J OF AUTOIMMUNITY. vol. 23, no. 2, September 2004, pages 151 - 160, XP004590159
- RACKE M K ET AL ANNALS OF NEUROLOGY. vol. 39, no. 1, 1996, pages 46 - 56, XP008021103
- HOLCOMBE H ET AL THE JOURNAL OF IMMUNOLOGY. vol. 169, 2002, pages 4982 - 4989, XP002491621
- SAKAGUCHI S. ET AL: 'Regulatory T Cells: Key Controllers of Immunologic Self-Tolerance' CELL, CELL PRESS, CAMBRIDGE, NA, US vol. 101, 26 May 2000, pages 455 - 458, XP003025450 ISSN: 0092-8674
- READ S. ET AL: 'CD4<+> regulatory T cells' CURRENT OPINION IN IMMUNOLOGY, ELSEVIER, OXFORD, GB vol. 13, no. 6, 01 December 2001, pages 644 - 649, XP004311242 ISSN: 0952-7915
- DATABASE BIOSIS [Online] BIOSCIENCES INFORMATION SERVICE, PHILADELPHIA, PA, US October 2002 LEWIS DAVID F.V.: 'Oxidative stress: The role of cytochromes P450 in oxygen activation.' Database accession no. PREV200300013366 & JOURNAL OF CHEMICAL TECHNOLOGY AND BIOTECHNOLOGY, vol. 77, no. 10, October 2002 (2002-10), pages 1095-1100, ISSN: 0268-2575
- DATABASE MEDLINE [Online] US NATIONAL LIBRARY OF MEDICINE (NLM), BETHESDA, MD, US November 2003 LAGE CLAUDIA ET AL: 'New insights on how nucleotide excision repair could remove DNA adducts induced by chemotherapeutic agents and psoralens plus UV-A (PUVA) in Escherichia coli cells.' Database accession no. NLM14644316 & MUTATION RESEARCH NOV 2003, vol. 544, no. 2-3, November 2003 (2003-11) , pages 143-157, ISSN: 0027-5107
- DATABASE BIOSIS [Online] BIOSCIENCES INFORMATION SERVICE, PHILADELPHIA, PA, US 2003 TAKEKOSHI SUSUMU ET AL: 'Involvement of lipid peroxidation in the alteration of protein kinase C signaling.' Database accession no. PREV200400079888 & ACTA HISTOCHEMICA ET CYTOCHEMICA, vol. 36, no. 4, 2003, pages 281-285, ISSN: 0044-5991
- MAURY C.P. ET AL: 'Comparative study of serum amyloid-related protein SAA, C-reactive protein, and beta 2-microglobulin as markers of renal allograft rejection' CLINICAL NEPHROLOGY, DUSTRI VERLAG, NUENCHEN-DEISENHOFEN, DE vol. 22, no. 6, 01 December 1984, pages 284 - 292, XP008095124 ISSN: 0301-0430
- Birgit Knoechel ET AL: "Sequential development of interleukin 2-dependent effector and regulatory T cells in response to endogenous systemic antigen", Journal of Experimental Medicine, vol. 202, no. 10, 21 November 2005 (2005-11-21), pages 1375-1386, XP55001304, ISSN: 0022-1007, DOI: 10.1084/jem.20050855
- ANONYMOUS: 'Ataxia telangiectasia' INTERNET CITATION, [Online] 11 June 2011, Wikipedia Retrieved from the Internet: <URL:http://en.wikipedia.org/wiki/Ataxia_te langiectasia> [retrieved on 2011-06-27]
- ANONYMOUS: 'Cockayne syndrome' INTERNET CITATION, [Online] 11 June 2011, Wikipedia Retrieved from the Internet: <URL:http://en.wikipedia.org/wiki/Cockayne_ syndrome> [retrieved on 2011-06-27]

## Description

### FIELD OF THE INVENTION

Numerous diseases have been linked to the production of effector cells. Disclosed herein is the realization that effector cells numbers are cycling in these diseases. In addition, disclosed herein is the determination that regulator cells are cycling in degenerative diseases. Based on these realizations, disclosed herein are uses of an agent in the manufacture of a medicament for treating conditions such as autoimmune diseases, degenerative diseases, and graft-versus-host disease. Also disclosed herein are methods of determining when therapy should be administered to a patient.

### BACKGROUND OF THE INVENTION

### Autoimmune diseases

Many autoimmune disorders arise when cells of specific tissues become the targets of T lymphocytes (for review see Santamaria, 2001). In some instances, T lymphocytes effect tissue damage directly through processes of cell-mediated cytotoxicity that involve Fas, perforin, or both. Perforin-mediated lysis requires a cognate interaction between the antigen-specific T cell receptor on a T lymphocyte and the specific antigen (usually a peptide) presented on an MHC molecule on the target cell's plasma membrane. Fas-mediated cytotoxicity involves the ligation of Fas on the target cell by Fas ligand (FasL) on T cells but does not require a cognate interaction between the effector lymphocyte and its target; and thus has the potential to damage innocent bystanders. In other instances, T lymphocytes kill their targets by secreting cytokines that can ligate pro-apoptotic receptors on the target cell. In other instances, autoreactive lymphocytes kill their targets indirectly, by enhancing their susceptibility to death-effector mechanisms mediated by non-lymphocytes, by promoting the recruitment of additional inflammatory cells into the target tissue (i.e. cytotoxic macrophages), or by driving the differentiation of autoreactive B cells into autoantibody-secreting plasma cells.

Much of what is currently known about effector pathways of autoimmunity has been learned from spontaneous and experimental models of autoimmune disease. Type 1 diabetes mellitus (T1D) in non-obese diabetic (NOD) mice is a prototypic model of spontaneous, organ-specific autoimmunity. NOD mice spontaneously develop a form of autoimmune diabetes, closely resembling human T1D, that results from destruction of the pancreatic β-cells by T lymphocytes.

Studies of CD8+-T-cell-deficient NOD mice indicate that the initial β-cell insult in T1D is effected by cytotoxic CD8+ T cells. Several transgenic models of T1D have shown that CD8+ T cells can readily kill β-cells expressing transgenic neo-antigens; however, little is known about the antigenic specificity or specificities of the CD8+ T cells that kill β-cells in NOD mice. Wong et al. (1999) have reported that there is a CD8+ T-cell subpopulation that recognizes an insulin-derived peptide in the islets of young NOD mice. Furthermore, immunopathological studies of pancreata from human diabetic individuals and pancreas isograft recipients have suggested that destruction of β-cells in human T1D may also be effected, at least in part, by CD8+ effector T cells (Bottazzo et al., 1985).

CD8+ effector T cells are also involved in the development of spontaneous autoimmune diseases of the thyroid. Hashimoto`s thyroiditis, for example, results from the destruction of thyroid follicular cells by CD8+ T cells (Bretz et al., 1999). It has also been reported that initiation of iodine-induced thyroiditis in NOD and NOD-H2h4 mice requires the contribution of CD8+ T cells (Verma et al., 2000). As in T1D, development of thyroid autoimmune diseases also involves CD4+ T cells.

Experimental autoimmune encephalomyelitis (EAE) is a prototypic experimental autoimmune disease that models human multiple sclerosis and that develops in susceptible rodent strains after immunization with myelin basic protein, proteolipid antigen or myelin oligodendrocyte protein (MOG). Evidence suggests that CD8+ T cells have a role in disease progression and severity (reviewed by Goverman, 1999). Myelin basic protein is processed *in vivo* by the MHC class I pathway, and a MOG-derived peptide activates encephalitogenic CD8+ T cells *in vivo*. There is also evidence for clonal expansions of CD8+ T cells in active multiple-sclerosis lesions (Babbe et al., 2000).

In many autoimmune disorders, T lymphocytes function as indirect effectors of autoimmunity by driving the differentiation of B lymphocytes into autoantibody-secreting plasma cells or by shedding autoantigens from target cells. These diseases include, among others, autoimmune hemolytic anemia, autoimmune thrombocytopenic purpura, Graves' disease, Goodpasture's syndrome, myasthenia gravis, pemphigus vulgaris and systemic lupus erythematosus.

### Graft-verslls-host disease

Graft-versus-host disease is a multistep process. It has been shown that effector T cells play the pivotal role in the induction of the disease. During the 'induction phase' the effector T cells see alloantigen disparities and then become activated and clonally expand (the 'expansion stage'). These T cells then release cytokines and possibly chemokines (for example macrophage inflammatory protein 1α), resulting in the recruitment of other cell types (macrophages, granulocytes, natural killer cells, etc.) in the 'recruitment phase'. Finally, the T cells and the other cell types mediate the pathology associated with graft-versus-host disease (the 'effector phase') (for a review see Murphy and Blazar, 1999).

There has been emphasis on delineating the effector mechanisms of graft-versus-host disease. T cells and other cells primarily mediate their effector functions through either FasL, perforin-granzyme-B or TNF. The recent use of knockout mice has demonstrated a pivotal role for each of these pathways in the effector stage of graft-versus-host disease. FasL and perforin-granzyme-B appear critical for solid organ pathology whereas TNF appears to mediate the wasting/weight loss associated with graft-versus-host disease. TNF also appears to be induced, along with other cytokines, after conditioning (Hill et al., 1997) - demonstrating that cytokines elicited by either the donor or the recipient affect graft-versus-host disease. TNF-receptor knockout mice and the use of anti-TNF antibodies have been shown to be protective in graft-versus-host disease models (Speiser et al., 1997).

### Degenerative diseases

Whilst degenerative diseases such as Alzheimer's disease are not classically considered to be mediated by inflammation or the immune system, in some instances the immune system may play an important role in the degenerative process. In addition, it has become clear that the immune system itself may have beneficial effects in nervous system diseases considered degenerative. Immunotherapeutic approaches designed to induce a humoral immune response have recently been developed for the treatment of Alzheimer's disease. In animal models, it has also been shown that immunotherapy designed to induce a cellular immune response may be of benefit in central nervous system injury, although T cells may have either a beneficial or detrimental effect depending on the type of T cell response induced (Monsonego and Weiner, 2003).

### Regulatory T cells

Studies have provided firm evidence for the existence of a naturally occurring population of regulatory/suppressor T cells, which, upon *in vitro* Tor-meditated stimulation, suppress the proliferation of effector T cells (von Herrath and Harrison, 2003). These cells are central to the control of T cell homeostasis and in the modulation of immune responses to autoantigens, cancer cells, pathogens, and alloantigens.

In the periphery of young mice not prone to autoimmune diseases, regulatory T cells constitute a stable 10% of CD4⁺ T cells. This proportion appears to be reduced in mice genetically prone to autoimmune disease such as diabetes (Salomon et al., 2000). Transfer of regulatory T cells has been shown to prevent a wide range of experimental autoimmune diseases, including diabetes, experimental autoimmune encephalomyelitis, and colitis (Salomon et al., 2000; Wu et al., 2002; Kohm et al., 2002; Read et al., 2000). Furthermore, depletion of regulatory T cells has been shown to exacerbate various experimental autoimmune diseases, including collagen induced arthritis (Morgan et al., 2003)). In humans, an analogous population of CD4⁺CD25⁺ regulatory T cells has been identified in the peripheral blood and the thymus (Jonuliet et al., 2001; Annunziato et al., 2002).

The potential for regulatory T cells to actively regulate autoimmune diseases and graft-versus-host disease, and induce long term tolerance has great potential application as a strategy for inducing long-lived tolerance. Taking advantage of regulatory T cells has been complicated by an inability to expand and characterize this minor T cell subset, a population of cells reduced even further in autoimmune-prone animals and patients. For instance, recent studies have suggested that it may be impossible to reverse ongoing autoimmune diabetes due to the autoreactive T cells becoming resistant to suppression during the active phase of the disease. Prior efforts to expand regulatory T cells *ex vivo* have not achieved clinically sufficient expansion, nor demonstrable *in vivo* efficacy (Fu et al., 2004). The low number of CD4+ CD25+ regulatory T cells, their anergic phenotype and diverse antigen specificity present major challenges to harnessing this potent tolerogenic population to treat autoimmune diseases and transplant rejection.

WO 2005/070090 provides methods for producing a predetermined autoantigen-specific regulatory T cell enriched composition, and resultant compositions and methods of use. In one example, WO 2005/070090 provides a method of modulating an autoimmune reaction in a subject by (a) obtaining a population of subject-compatible cells; (b) producing an autoantigen-specific, preferably predetermined autoantigen-specific regulatory T cell enriched composition from said population of cells; and (c) introducing said composition into said subject to modulate said autoimmune reaction in said subject.

### Acute phase inflammatory markers

Measurements of certain acute-phase protein plasma concentrations can be of diagnostic or prognostic value under specific clinical conditions. The best known acute-phase protein is C-reactive protein (CRP). CRP is a plasma protein that rises in the blood with the inflammation from certain conditions. The level of CRP in blood plasma can rise as high as 1000-fold with inflammation. Conditions that commonly lead to marked changes in CRP include bacterial and viral infection, trauma, surgery, burns, inflammatory conditions, coronary and vascular disease and advanced cancer.

Most acute phase proteins are synthesized by hepatocytes, some are produced by other cell types, including monocytes, endothelial cells, fibroblasts and adipocytes. Acute phase proteins include serum amyloid A (SAA), CRP and serum amyloid P component (SAP).

The immediate responsiveness of CRP and SAA to stimuli, together with their wide concentration range and ease of automated measurement, have led to plasma CRP and SAA levels being used to monitor accurately the severity of inflammation and the efficacy of disease management during certain disease conditions.

J. Immunol., 2002, 169:4982-4989 discloses that the immunosuppressant 15-deoxyspergualin functions by inhibiting CD4+ T cell expansion. Arch. Neurol., 1997, 54:485-8 discloses an improvement in dementia following cytotoxic therapy.

Previous studies do not appreciate that the immune system, including effector cell populations, are cycling (oscillating in numbers) in a repetitive and differential manner in autoimmune diseases and during transplantation rejection. Further more, prior studies do not appreciate that the immune response, including regulator cell populations, are cycling in degenerative disease states. Disclosed herein is the realization of these cycles, and thus uses of an agent in the manufacture of a medicament for the treatment of these diseases.

### SUMMARY OF THE INVENTION

The present inventors have surprisingly found that both effector cell and regulator cell numbers cycle during disease states characterized by the presence of effector cells. This cycling occurs on a regular basis, with effector cell expansion against a target antigen being followed by the expansion of regulator cells directed against the effectors. Upon control of the effector cells by the regulator cells the numbers of both types of cells decrease, which in turn is followed by the same cycle due to the continuous presence of antigen.

Knowledge of this cycle can be used to treat diseases where it is known that the emergence of effector cells is detrimental to the patient. Examples of such conditions are autoimmune diseases and transplantation rejection. More specifically, treatment of a patient can be timed such that effector cell expansion does not occur, and/or effector cell number are reduced or abolished.

Thus, in a first aspect, the present invention provides a method or use as defined in the appended claims.

With regard to autoimmune diseases, whilst not wishing to be limited by theory, it appears that effector cell expansion against a target self antigen is followed by the expansion of regulator cells directed against the effectors. Upon control of the effector cells by the regulator cells the numbers and/or activity of both types of cells decrease, which in turn is followed by the same cycle due to the continuous effort of the patients immune system to target self antigens.

Preferably, the immune system marker is an acute phase inflammatory marker. More preferably, the acute phase inflammatory marker is selected from the group consisting of serum amyloid A, serum amyloid P and c-reactive protein.

Preferably, the agent is administered between when the levels of an acute phase inflammatory marker have reached their lowest point and before the marker peaks in the next cycle.

Preferably, the immune system marker reflects the number and/or activity of regulator cells, and/or the number and/or activity of effector cells.

In another embodiment, the immune system marker is body temperature. With respect to this embodiment, it is preferred that the agent is administered when body temperature has reached its lowest point and before body temperature peaks in the next cycle.

Preferably, the effector T cells are CD8+CD4- T cells. Preferably, the agent is administered between when CD8+CD4- T cells numbers are at their lowest point and before CD8+CD4- T cells numbers peak in the next cycle. More preferably, the agent is administered about when CD8+CD4- T cells numbers are at their lowest point cycle or begin increasing in the next cycles.

Preferably, the regulator T cells are CD4+CD8- T cells. Preferably, the agent is administered approximately when, or just before, CD4+CD8- T cell numbers have peaked.

With regard to the above aspect, the agent inhibits the production of, limits the function of, and/or destroys; effector cells and the agent is selected from the group consisting of anti-proliferative drugs, anti-metabolic drugs, radiation, dsRNA and antibodies which inhibit the production, limit the function of and/or activity of effector cells. Preferably, the anti-proliferative drug is selected from the group consisting of: taxol, vincristine, vinblastine and anhydro vinblastine.

An example of a preferred antibody is anti-CD8+ antibody.

The observation that the immune system is cycling during disease states characterized by the presence of effector cells can also be used as an indicator of the presence of such a disease. These diagnostic procedures would be particularly useful for analysing a patient for the recurrence of the disease state (such as autoimmune disease) following treatment, or for analysing a patient determined to be susceptible to the disease (such as in cases where the subject has previously been identified as possessing an allele of a gene which predisposes the subject to an autoimmune disease) for the emergence of the disease.

Also disclosed herein is a method for analysing effector cell and/or regulator cell cycling to diagnose a disease characterized by the production of effector cells, the method comprising monitoring the patient, or samples obtained therefrom, for fluctuations in at least one of: a) effector cell numbers and/or activity, b) regulator cell numbers and/or activity, c) a molecule associated with the disease, and/or d) an immune system marker, wherein cycling of any one of a) to d) indicates the disease may be present.

Also disclosed herein is the use of an assay which detects an immune system marker for analysing effector cell and/or regulator cell cycling to determine when an agent should be administered to a patient suffering from a disease characterized by the production of effector cells.

Preferably, the marker is an acute phase inflammatory marker. More preferably, the acute phase inflammatory marker is selected-from the group consisting of: serum amyloid A, serum amyloid P and c-reactive protein.

Also disclosed herein is the use of an assay which detects effector cell numbers and/or activity for analysing effector cell and/or regulator cell cycling to determine when an agent should be administered to a patient suffering from a disease characterized by the production of effector cells.

Preferably, the assay detects the number of CD8+CD4- T cells.

Also disclosed herein is the use of an assay which detects regulator cell numbers and/or activity for analysing effector cell and/or regulator cell cycling to determine when an agent should be administered to a patient suffering from a disease characterized by the production of effector cells.

Preferably, the assay detects the number of CD4+CD8- T cells.

Also disclosed herein is the use of an assay which detects a molecule associated with a disease characterized by the production of effector cells for analysing effector cell and/or regulator cell cycling to determine when an agent should be administered to treat the disease.

Also disclosed herein is a kit when used for analysing effector cell and/or regulator cell cycling to determine when an agent should be administered to a patient suffering from a disease characterized by the production of effector cells, the kit comprising at least one reagent for monitoring the patient, or samples obtained therefrom, for fluctuations in at least one of: a) effector cell numbers and/or activity, b) regulator cell numbers and/or activity, c) a molecule associated with the disease, and/or d) an immune system marker.

Preferably, the kit comprises written instructions for performing a method of the invention including reference to the preferred number of samples to be analysed, and the timing between sample analysis.

The present inventors have also surprisingly found that both effector cell and regulator cell numbers are cycling during a degenerative disease. This cycling occurs on a regular basis, with effector cell expansion against a target antigen being followed by the expansion of regulator cells directed against the effectors. Upon control of the effector cells by the regulator cells the numbers of both types of cells decrease, which in turn is followed by the same cycle due to the continuous presence or incomplete removal of antigen.

Knowledge of this cycle can be used to treat diseases where it is known that the emergence of regulator cells is detrimental to the patient. More specifically, treatment of a patient can be timed such that regulator cell expansion does not occur, and/or regulator cell numbers are reduced or abolished.

Examples of degenerative diseases include, Alzheimer's disease, and a prion related disease.

Whilst not wishing to be limited by theory, it appears that effector cell expansion against a target antigen is followed by the expansion of regulator cells directed against the effectors. Whilst not traditionally considered as diseases characterized by the production of an antigen, degenerative diseases such as Alzheimer's disease and a prion related disease are caused, at least in part, by elevated levels of a particular protein(s) (antigen). There is evidence that an immune response can be raised against such proteins, and some suggestion has been made to producing vaccines to treat degenerative disease such as Alzheimer's disease and a prion related diseases. Upon control of the effector cells by the regulator cells the numbers and/or activity of both types of cells decrease, which in turn is followed by the same cycle due to the continuous presence or incomplete removal of antigen which results in an oscillating persistent, but ineffective, immune response.

An appropriate time to administer the agent is between when the levels of acute phase inflammatory marker have peaked and before the marker begins to rise in the next cycle. Accordingly, a particularly preferred immune system marker is an acute phase inflammatory marker. More preferably, the acute phase inflammatory marker is selected from, but not limited to, the group consisting of serum amyloid A, serum amyloid P and c-reactive protein.

Preferably, the immune system marker reflects the number and/or activity of regulator cells, and/or the number and/or activity of effector cells.

In another embodiment, the immune system marker is body temperature. With respect to this embodiment, it is preferred that the agent is administered when body temperature has peaked and before body temperature begins to rise in the next cycle.

In one embodiment, the patient is monitored for an increase in the number and/or activity of regulator cells by the analysis of CD4+CD8- T cell levels. With regard to this embodiment, it is preferred that the agent is administered between when CD4+CD8- T cells numbers are at their lowest point and before CD4+CD8- T cells numbers peak in the next cycle. More preferably, the agent is administered about when CD4+CD8- T cells numbers are at their lowest point or begin increasing in the next cycle.

In another embodiment, the patient is monitored for an increase in the number and/or activity of effector cells by the analysis of CD8+CD4- T cell levels. With regard to this embodiment, it is preferred that the agent is administered approximately when, or just before, CD8+CD4- T cell numbers have peaked.

With regard to aspects of the invention that relate to degenerative diseases, the agent inhibits the production of, limits the function of; and/or destroys, regulator cells and the agent is selected from the group consisting of anti-cancer drugs such as anti-proliferative drugs, anti-metabolic drugs, radiation, dsRNA and antibodies which inhibit the production and/or activity of regulator cells. Preferably, the anti-proliferative drug is selected from the group consisting of, but not limited to, taxol, vincristine, vinblastine and anhydro vinblastine. Examples of preferred antibodies include, but are not limited to, anti-CD4+, anti-CTLA-4 (cytotoxic lymphocyte-associated antigen-4), anti-GITR (glucocorticoid-induced tumour necrosis factor receptor), anti-CD28 and anti-CD25.

The observation that the immune system is cycling during a degenerative disease can also be used as an indicator of the presence of such a disease. These diagnostic procedures would be particularly useful for analysing a patient for the recurrence of the disease state following treatment, or for analysing a patient determined to be susceptible to the disease for the emergence of the disease.

Also disclosed herein is a method for analysing effector cell and/or regulator cell cycling to diagnose a degenerative disease, the method comprising monitoring the patient, or samples obtained therefrom, for fluctuations in at least one of: a) effector cell numbers and/or activity, b) regulator cell numbers and/or activity, c) a molecule associated with the disease, and/or d) an immune system marker, wherein cycling of any one of a) to d) indicates the disease may be present.

The present inventors have also determined that treatment for a degenerative disease can be enhanced (or the chances of successful treatment can be increased) when a vaccine is administered at the appropriate time. In these instances, the vaccine boosts the innate immune response against the disease. This will most likely be a result of increased numbers and/or activity of effector cells. Although theoretically regulator cells will still ultimately be produced, the boosting of the immune system allows the patient to suitably control the disease before the emergence of the regulator cells.

In one embodiment, the vaccine is administered about when the levels of effector cells are increasing. In another embodiment, the vaccine is administered about when the levels of a molecule associated with the disease begin to decrease. In a further embodiment, the vaccine is administered about when the levels of an acute phase inflammatory marker begin to increase.

Also disclosed herein is the use of an assay which detects an immune system marker for analysing effector cell and/or regulator cell cycling to determine when an agent or vaccine should be administered to a patient suffering from a degenerative disease.

Preferably, the marker is an acute phase inflammatory marker. More preferably, the acute phase inflammatory marker is selected from the group consisting of: serum amyloid A, serum amyloid P and c-reactive protein.

Also disclosed herein is the use of an assay which detects effector cell numbers and/or activity for analysing effector cell and/or regulator cell cycling to determine when an agent or vaccine should be administered to a patient suffering from a degenerative disease.

Preferably, the assay detects the number of CD8+CD4- T cells.

Also disclosed herein is the use of an assay which detects regulator cell numbers and/or activity for analysing effector cell and/or regulator cell cycling to determine when an agent or vaccine should be administered to a patient suffering from a degenerative disease.

Preferably, the assay detects the number of CD4+CD8- T cells.

Also disclosed herein is the use of an assay which detects a molecule associated with a degenerative disease for analysing effector cell and/or regulator cell cycling to determine when an agent or vaccine should be administered to treat the disease.

Also disclosed herein is the use of an agent for the manufacture of a medicament for administering to a patient suffering from a degenerative disease, wherein the agent will be administered at a time selected such that the activity of effector cells is not significantly reduced.

Preferably, the agent inhibits the production of, limits the function of, and/or destroys, regulator cells.

Also disclosed herein is a kit when used for analysing effector cell and/or regulator cell cycling to determine when an agent or vaccine should be administered to a patient suffering from a degenerative disease, the kit comprising at least one reagent for monitoring the patient, or samples obtained therefrom, for fluctuations in at least one of: a) effector cell numbers and/or activity, b) regulator cell numbers and/or activity, c) a molecule associated with the disease, and/or d) an immune system marker.

Preferably, the kit comprises written instructions for performing a method of the invention including reference to the preferred number of samples to be analysed, and the timing between sample analysis.

As outlined herein, the present inventors have noted that fluctuations in numerous factors indicate that the immune system is cycling in patients suffering from a disease characterized by the production of effector cells, as well as degenerative diseases. These factors include acute phase inflammatory markers. These factors are linked, directly or indirectly, to the general state of the immune system including, but not necessarily limited to, effector cell production and/or activity, regulator cell production and/or activity, and/or B cell production and/or activity.

It will be appreciated by the skilled person that diseases such as autoimmune diseases and degenerative diseases have a complex effect on the patient. Furthermore, natural variations between individuals linked to factors such as their genotype, nutrition, fitness, previous and current disease status, all influence how a given individual responds to a disease state. Thus, individuals will vary in the periodicity or wavelength or frequency of their immune system cycle depending on their disease state. In addition, like the menstrual cycle, the length of the cycle may vary slightly within an individual due to natural variation and/or environmental factors. Thus, individual variation may at least be encountered with regard to, for example, i) the length of the cycle, ii) the absolute numbers of effector or regulator cells during the cycle, or iii) the levels of acute phase inflammatory markers during the cycle. Such variation may be exaggerated in patients with advanced disease, where the patient's immune system has been challenged for a considerable length of time.

As result, it will most likely be desirable to monitor the patient for a sufficient length of time to follow trends in fluctuations between and within cycles, and hence sufficient to determine the maximum and/or minimum, whichever is required for the relevant cell type, or marker thereof. This ensures that the dynamics of the immune system cycling within a particular patient is understood. Preferably, the patient is monitored for a period of at least 7 days, more preferably at least 14 days, more preferably at least 21 days, more preferably at least 28 days. It may also be desirable to monitor the patient for longer periods such as at least 35 days, at least 42 days, at least 49 days, or even longer.

An exception to monitoring the patient for a reasonable length of time is when the method is used to treat a transplantation rejection. In this case, exposure to effector cells may quickly result in rejection of the graft. Thus, when dealing with, for example, graft-versus-host disease it is preferred that monitoring of the patients begins immediately, or soon after, receiving the graft, and effector cells targeted during the first immune cycle. For example, in a preferred embodiment monitoring begins about 1 day after receiving the graft and continues for at least 15 days, more preferably at least 21 days.

In general, it is preferred that numerous factors are monitored at the same time. This is because, due to the factors describe above, it is unlikely that each factor will have a perfect cycle profile, particularly over a number of cycles, to routinely provide a clear indication of the appropriate time to administer the agent. Whilst the analysis of numerous factors over a long period may be costly, and may be of at least some inconvenience to the patient, diseases such as autoimmune diseases and degenerative diseases can be life threatening. Hence, it is worthwhile understanding as much as possible regarding immune system cycling in a given patient before the patient is treated.

In addition, although the analysis of different factors cycling in some patients may result in complex profiles, given the guidance provided herein it is well within the skill of the medical practitioner to analyse the monitoring data to determine the optimal time to administer the agent.

A further complicating factor will be if the patient has recently acquired a disease or trauma unrelated to that being treated. For example, a patient being treated for an autoimmune disease may also contract the common flu virus. The presence of the flu virus will result in, for example, an increase in acute phase inflammatory markers independent of the cycling of these markers which is occurring due to the autoimmune disease. Other diseases which may cause complications in monitoring effector/regulator cell cycling for use in the methods of the present invention include, other infections, and cancer. Accordingly, it is desirable to monitor the patient for any factors which may result in elevated levels of, for example, acute phase inflammatory markers to ensure that the factor being monitored truly reflects effector/regulator cell cycling resulting from the disease being treated.

Furthermore, it is preferred that the patient is monitored as frequently as possible to ensure immune system cycling within a given patient is suitably characterized. Naturally, this will ensure that the agent is administered at the appropriate time and that any small variations in, for example, effector/regulator cell numbers or activity, or markers thereof, is not misinterpreted. Preferably, the patient is monitored at least every 3 days, more preferably at least every 2 days, and most preferably at least every day. Monitoring may occur more frequently, for instance every 12 hours, when the cycling is reaching a stage where it is likely that the timing would be appropriate to administer the agent.

Preferably, the patient is a mammal. More preferably, the mammal is a human.

As will be apparent, preferred features and characteristics of one aspect of the invention are applicable to many other aspects of the invention.

Throughout this specification the word "comprise", or variations such as "comprises" or "comprising", will be understood to imply the inclusion of a stated element, integer or step, or group of elements, integers or steps, but not the exclusion of any other element, integer or step, or group of elements, integers or steps.

The invention is hereinafter described by way of the following non-limiting Examples and with reference to the accompanying figures.

### BRIEF DESCRIPTION OF THE ACCOMPANYING DRAWINGS

Figure 1. C-reactive Protein and Serum Amyloid A versus time in Mrs FO.
Figure 2. Serum Amyloid A and IL-2 versus time in Mrs FO.
Figure 3. Serum Amyloid A and cancer marker CA125 versus time in Mrs FO.
Figure 4. C-reactive Protein and C3 versus time in Mrs FO.

### DETAILED DESCRIPTION OF THE INVENTION

### General Techniques and Definitions

Unless specifically defined otherwise, all technical and scientific terms used herein shall be taken to have the same meaning as commonly understood by one of ordinary skill in the art (e.g., in cell culture, molecular genetics, immunology, immunohistochemistry, protein chemistry, and biochemistry).

Unless otherwise indicated, the recombinant protein, cell culture, and immunological techniques utilized in the present invention are standard procedures, well known to those skilled in the art. Such techniques are described and explained throughout the literature in sources such as, J. Perbal, A Practical Guide to Molecular Cloning, John Wiley and Sons (1984), J. Sambrook et al., Molecular Cloning: A Laboratory Manual, Cold Spring Harbour Laboratory Press (1989), T.A. Brown (editor), Essential Molecular Biology: A Practical Approach, Volumes 1 and 2, IRL Press (1991), D.M. Glover and B.D. Hames (editors), DNA Cloning: A Practical Approach, Volumes 1-4, IRL Press (1995 and 1996), and F.M. Ausubel et al. (editors), Current Protocols in Molecular Biology, Greene Pub. Associates and Wiley-Interscience (1988, including all updates until present), Ed Harlow and David Lane (editors) Antibodies: A Laboratory Manual, Cold Spring Harbour Laboratory, (1988), and J.E. Coligan et al. (editors) Current Protocols in Immunology, John Wiley & Sons (including all updates until present).

As used herein the terms "treating", "treat" or "treatment" include administering a therapeutically effective amount of an agent sufficient to reduce or eliminate at least one symptom of the disease.

"Effector cells" include, but are not necessarily limited to, the T cell population known as CD8+ cells.

"Regulator cells" include, but are not necessarily limited to, a subpopulation of CD4+ T cells. Such cells may also be referred to in the art as "suppressor cells". Regulator cells may either act directly on effector cells or may assert their affects upon effector cells through other mechanisms.

CD4+ cells express the marker known in the art as CD4. Typically, the term "CD4+ T cells" as used herein does not refer to cells which also express CD8. However, this term can include T cells which also express other antigenic markers such as CD25.

As used herein, the term "inhibits the production of, limits the function of, and/or destroys" when referring to the exposure of the "effector cells" to the agent means that the number, and/or activity, of effector cells is down-regulated by the agent. Most preferably, the number, and/or activity, of effector cells is completely eradicated by the agent.

As used herein, the term "inhibits the production of, limits the function of, and/or destroys" when referring to the exposure of the "regulator cells" to the agent means that the number, and/or activity, of regulator cells is down-regulated by the agent. Most preferably, the number, and/or activity, of regulator cells is completely eradicated by the agent.

As used herein the term "disease characterized by the production of effector cells" refers to any condition wherein the number or activity of effector cells plays a role in prolonging the disease state. These disease are either i) typically characterized by an immune response against self antigens known generally in the art as autoimmune diseases, or ii) involve a patients immune response during organ/tissue/cell transplantation from a suitable donor.

As used herein, the term "autoimmune disease" refers to any disease in which the body produces an immunogenic (ie, immune system) response to some constituent of its own tissue. In other words the immune system loses its ability to recognize some tissue or system within the body as "self" and targets and attacks it as if it were foreign. Autoimmune diseases can be classified into those in which predominantly one organ is affected (eg, hemolytic anemia and anti-immune thyroiditis), and those in which the autoimmune disease process is diffused through many tissues (eg, systemic lupus erythematosus). Examples of autoimmune diseases include, but are not limited to, rheumatoid arthritis, multiple sclerosis, lupus erythematosis, myasthenia gravis, scleroderma, Crohn's disease, ulcerative colitis, Hashimoto's disease, Graves' disease, Sjogren's syndrome, polyendocrine failure, vitiligo, peripheral neuropathy, autoimmnune polyglandular syndrome type I, acute glomerulonephritis, Addison's disease, adult-onset idiopathic hypoparathyroidism (AOIH), alopecia totalis, amyotrophic lateral sclerosis, ankylosing spondylitis, autoimmune aplastic anemia, autoimmune hemolytic anemia, Behcet's disease, Celiac disease, chronic active hepatitis, CREST syndrome, dermatomyositis, dilated cardiomyopathy, eosinophiliamyalgia syndrome, epidermolisis bullosa acquisita (EBA), giant cell arteritis, Goodpasture's syndrome, Guillain-Barr syndrome, hemochromatosis, Henoch-Schonlein purpura, idiopathic IgA nephropathy, insulin-dependent diabetes mellitus (IDDM), juvenile rheumatoid arthritis, Lambert-Eaton syndrome, linear IgA dermatosis, myocarditis, narcolepsy, necrotizing vasculitis, neonatal lupus syndrome (NLE), nephrotic syndrome, pemphigoid, pemphigus, polymyositis, primary sclerosing cholangitis, psoriasis, rapidly-progressive glomerulonephritis (RPGN), Reiter's syndrome, stiff-man syndrome, inflammatory bowel disease, osteoarthritis and thyroiditis.

The term "transplant" and variations thereof refers to the insertion of a graft into a host, whether the transplantation is allogeneic (where the donor and recipient are of different genetic origins but of the same species), or xenogeneic (where the donor and recipient are from different species). Thus, in a typical scenario, the host is human and the graft is an isograft, derived from a human of the same or different genetic origins. In another scenario, the graft is derived from a species different from that into which it is transplanted, such as a baboon heart transplanted into a human recipient host, and including animals from phylogenically widely separated species, for example, a pig heart valve, or animal beta islet cells or neuronal cells transplanted into a human host. Cells, tissues and/or organs may be transplanted, examples include, but are not limited to, isolated cells such as islet cells; tissue such as the amniotic membrane of a newborn, bone marrow, hematopoietic precursor cells, and ocular tissue, such as corneal tissue; and organs such as skin, heart, liver, spleen, pancreas, thyroid lobe, lung, kidney, tubular organs (e.g., intestine, blood vessels, or esophagus), etc. The tubular organs can be used to replace damaged portions of esophagus, blood vessels, or bile duct. The skin grafts can be used not only for burns, but also as a dressing to damaged intestine or to close certain defects such as diaphragmatic hernia. The graft is derived from any mammalian source, including human, whether from cadavers or living donors. Preferably the graft is bone marrow or an organ such as heart.

As used herein, "transplant rejection" or variations thereof refers to the host's immune system mounting an immune response to the graft, ultimately resulting in the graft being rejected by the host. There are generally two types of "transplant rejection", namely graft-versus-host disease and host-versus-graft disease.

As used herein, the term "graft-versus-host disease" refers to is an immune attack on the recipient by cells from a donor, often leading to rejection of the transplanted cells. Whilst the transplanted cells can be of any cell type, typically the only transplanted tissues that house enough immune cells to cause graft versus host disease are the blood and the bone marrow.

As used herein, the term "host-versus-graft disease" refers to the lymphocyte-mediated reactions of a host against allogeneic or xenogeneic cells acquired as a graft or otherwise, which lead to damage or/and destruction of the grafted cells. This is the common basis of graft rejection.

As used herein, a "degenerative disease" is a condition that results in the loss of cells. Preferably, the degenerative disease is a neurodegenerative disease which is marked by the loss of nerve cells. Neurodegenerative diseases relevant to the present invention are Alzheimer disease and prion related diseases such as Creutzfeldt-Jakob disease, Alper's disease, Kuru, Gersymann-Straussler-Scheinker syndrome, Fatal familial insomnia, scrapie, transmissible milk encephalopathy, chronic wasting disease, and bovine spongiform encephalopathy. In another embodiment, the degenerative disease is an "amyloid related disease" and is Alzheimer disease.

The term "immune system marker" generally refers to any molecule or factor which provides an indication of the state and/or activity of the immune system. These markers may be directly linked to the activity and/or production of regulator and/or effector cells, and/or may provide a more general indication of the overall response of the immune system to an antigen. Examples of a suitable immune system marker include acute phase inflammatory markers such as c-reactive protein and serum amyloid A. Another example of an immune system marker are indicators of cellular destruction such as, but not limited to, cholesterol and β-2-microglobulin in serum. Cholesterol and β-2-microglobulin are integral components of cellular membranes. In particular, β-2-microglobulin is the accessory molecule to the Major Histocompatabilty Class I or MHC- I receptor. Consequently, with the cycling of the anti-disease immune response together with target cell destruction, the serum levels in diseased patients of these two molecules is often elevated. Thus, oscillations in indicators of cellular destruction, such as cholesterol and β-2-microglobulin, may also prove useful in determining the beginning or end of the immune response cycle. Naturally, upon the present discovery of the immune system cycling in a disease characterized by the production of effector cells, as well as cycling in patients with a degenerative disease, the skilled addressee could readily identify further markers useful in the methods of the invention.

As used herein, the term "a molecule associated with the disease" refers to any molecule which is linked to the disease state. In a preferred embodiment, the marker is a protein. Such protein markers are well known in the art. For example, levels of amyloid-β peptide can be a marker of Alzheimer's disease, and prion proteins in their β-confirmation can be a marker of prion related diseases.

As used herein, the term "the activity of the effector cells is not significantly reduced" means that the timing of the administration of the agent (for treating a degenerative disease) is such that the agent exerts a proportionally greater effect against the regulator cells than the effector cells. It is clearly preferred that the agent is administered at a time when the ratio of effect against the regulator cells to the effect against effector cells is greatest.

As used herein, "cycling" or "cycle" or variations thereof refers to a repetitive oscillation of an indicator (cell number, activity of, marker of disease, immune system marker etc.), wherein the indicator changes periodically from a maximum to a minimum.

As used herein, the term "analysing effector cell and/or regulator cell cycling" refers to the determination of the defined cells, markers and/or molecules to gain an appreciation of the stage of the cycle at any given time. Preferably, the number of time points analysed, the period between each analysis, and the length of time the analysis is performed is sufficient to determine when the agent should be administered.

The "sample" refers to a material suspected of containing regulator cells, effectors cells, immune system markers and/or a molecule associated with the disease. The sample can be used as obtained directly from the source or following at least one step of (partial) purification. The sample can be prepared in any convenient medium which does not interfere with the method of the invention. Typically, the sample is an aqueous solution or biological fluid as described in more detail below. The sample can be derived from any source, such as a physiological fluid, including blood, serum, plasma, saliva, sputum, ocular lens fluid, buccal swab, sweat, faeces, urine, milk, ascites fluid, mucous, synovial fluid, peritoneal fluid, transdermal exudates, pharyngeal exudates, bronchoalveolar lavage, tracheal aspirations, cerebrospinal fluid, semen, cervical mucus, vaginal or urethral secretions, amniotic fluid, and the like. Preferably, the sample is blood or a fraction thereof. Pretreatment may involve, for example, preparing plasma from blood, diluting viscous fluids, and the like. Methods of treatment can involve filtration, distillation, separation, concentration, inactivation of interfering components, and the addition of reagents. The selection and pretreatment of biological samples prior to testing is well known in the art and need not be described further.

For the purposes of this invention, the term "antibody", unless specified to the contrary, includes fragments of whole antibodies which retain their binding activity for a target analyte. Such fragments include Fv, F(ab') and F(ab')₂ fragments, as well as single chain antibodies (scFv). Furthermore, the antibodies and fragments thereof may be humanised antibodies, for example as described in EP-A-239400.

### Acute Phase Inflammatory Markers

Some acute phase inflammatory markers initially increase during an immune response (referred to hereinafter as positive acute phase inflammatory markers) whilst others initially decrease during an immune response (referred to hereinafter as negative acute phase inflammatory markers). Acute phase inflammatory markers are also referred to in the art as acute phase reactants or acute phase proteins. The skilled addressee will be aware of the many assays which can be used to monitor acute phase inflammatory markers.

Examples of positive acute phase inflammatory markers include, but are not limited to, c-reactive protein, serum amyloid A, serum amyloid P component, complement proteins such as C2, C3, C4, C5, C9, B, C1 inhibitor and C4 binding protein, fibrinogen, von Willebrand factor, al-antitrypsin, α1-antichymotrypsin, α2-antiplasmin, heparin cofactor II, plasminogen activator inhibitor I, haptoglobin, haemopexin, ceruloplasmin, manganese superoxide dismutase, α1-acid glycoprotein, haeme oxygenase, mannose-binding protein, leukocyte protein I, lipoporotein (a), lipopolysaccharide-binding protein, and interleukins such as IL-1, IL-2, IL-6, IL-10 and receptors thereof.

Example of negative acute phase inflammatory markers include, but are not limited to, albumin, pre-albumin, transferin, apoAI, apoAII, α2 HS glycoprotein, inter-α-trypsin inhibitor, histidine-rich glycoprotein.

Serum amyloid A (SAA) was discovered as a plasma component that shares antigenicity with amyloid AA, the chief fibrillar component in reactive AA amyloid deposits. SAA has been shown to be an acute phase reactant whose level in blood is elevated to 1000-fold or higher as part of the body's responses to various injuries including trauma, infection and inflammation.

SAA levels can be determined as known in the art, see for example Weinstein *et al* (1984), Liuzzo *et al* (1994), O'Hara *et al* (2000), Kimura *et al* (2001) and O'Hanlon *et al* (2002).

C-reactive protein (CRP) is an important positive acute phase response protein, and its concentration in serum may increase as much as 1,000-fold during the acute phase response. CRP is a pentamer consisting of five identical subunits, each having a molecular weight of about 23,500.

C-reactive protein levels can be determined using techniques known in the art, these include, but are not limited to, those disclosed in Senju *et al* (1983), Weinstein *et al* (1984), Price *et al* (1987), Liuzzo *et al* (1994), Eda *et al* (1998), Kimura *et al* (2001) and O'Hanlon *et al* (2002).

The complement proteins are a group of at least 20 immunologically distinct components. They normally circulate in the blood in an inactive form. They are able to interact sequentially with antigen - antibody complexes, with each other and with cell membranes in a complex but adaptable way to destroy viruses and bacteria and pathologically, even the hosts own cells. Abnormal serum levels of complement proteins may be due to either inherited or acquired diseases. At least circulating levels of C3 and C4 reflect a balance between complement consumption due to immune complex formation and increased synthesis due to acute phase response. Methods of measuring complement protein levels are well known in the art.

Levels of different interleukins can also be determined using procedures known in the art such as using the ProteoPlex^{™} cytokine assay kit (EMD Biosciences Inc., CA, USA).

### Agents

The present disclosure relates broadly to the use of three different types of agents. There are:
1) agents which are specific for effector cells (such as CD8+ specific antibodies) that can be used to treat a disease characterized by the production of effector cells,
2) agents which are specific for regulator cells (such as CD4+ specific antibodies) that can be used to treat a degenerative disease, and
3) non-selective agents which influence effector cells and regulator cells, however, the timing of administration of the agent dictates the cell type being targeted.

### Agents for treating a disease characterized by the production of effector cells

The agent can be any factor or treatment which selectively or non-selectively results in the destruction, limits the function of, or the inhibition of the production, of effector cells. For example, a CD8+ specific antibody could be used to specifically target CD8+ T cells. However, in some instances a non-selective agent could be used, such as an anti-proliferative drug, an anti-metabolic drug or radiation, each of which traget dividing cells. In particular, as with other cell types, effector cells are particularly vulnerable to destruction by anti-mitotic (anti-proliferative) drugs or spindle poisons (e.g. vinblastine or paclitaxel) when dividing and specifically in mitosis.

The term "anti-proliferative drug" and "anti-metabolic drug" is a term well understood in the art and refers to any compound that destroys dividing cells or inhibits them from undergoing further proliferation. Anti-proliferative drugs include, but are not limited to, mechlorethamine, cyclophosphamide, ifosfamide, melphalan, chlorambucil, hexamethyl-melamine, thiotepa, busulfan, carmustine, lomustine, semustine, streptozocin, dacarbazine, methotrexate, fluorouracil, floxuridine, cytarabine, mercaptopurine, thioguanine, pentostatin, vinblastine, anhydro vinblastine, vincristine, etoposide, teniposide, dactinomycin, daunorubicin, doxorubicin, bleomycin, plicamycin, mitomycin, L-asparaginase, cisplatin, mitoxantrone, hydroxyurea, procarbazine, mitotane, aminoglutethimide, prednisone, hydroxyprogesterone caproate, medroprogesterone acetate, megestrol acetate, diethylstilbestrol, ethinyl estradiol, tamoxifen, testosterone propionate, radioactive isotopes, ricin A chain, taxol, diphtheria toxin, colchicine and pseudomonas exotoxin A.

The agents are usually administered in the dosage forms that are readily available to the skilled clinician, and are generally administered in their normally prescribed amounts (as for example, the amounts described in the Physician's Desk Reference, 55th Edition, 2001, or the amounts described in the manufacture's literature for the use of the agent).

In one embodiment, the agent is administered as a single bolus injection. In another embodiment, the agent is administered by infusion. The period of infusion can be, for example, at least 3 hours, at least 12 hours or at least 24 hours.

Another example of an agent which can be administered in a method of the invention is dsRNA. dsRNA is used in RNA interference (RNAi) which is a phenomenon where upon introduction into a cell, mRNA homologous to the dsRNA is specifically degraded so that synthesis of gene products is suppressed. Examples of such an agent causing RNAi include, but are not limited to, a sequence having at least about 70% homology to the nucleic acid sequence of a target gene or a sequence hybridizable under stringent conditions, RNA containing a double-stranded portion having a length of at least 10 nucleotides or variants thereof. Examples of target genes include, but are not limited to, a gene required for replication or survival of a effector cell.

dsRNA having a length of about 20 bases (e.g., representatively about 21 to 23 bases) or less than about 20 bases, which is called siRNA in the art, can be used. Expression of siRNA in cells can suppress expression of a gene targeted by the siRNA. In another embodiment, an agent capable of causing RNAi may have a short hairpin structure having a sticky portion at the 3' terminus (shRNA; short hairpin RNA). As used herein, the term "shRNA" refers to a molecule of about 20 or more base pairs in which a single-stranded RNA partially contains a palindromic base sequence and forms a double-strand structure therein (i.e., a hairpin structure). shRNA can be artificially chemically synthesized. Alternatively, shRNA can be produced by linking sense and antisense strands of a DNA sequence in reverse directions and synthesizing RNA *in vitro* with T7 RNA polymerase using the DNA as a template. The length of the double-stranded portion is not particularly limited, but is preferably about 10 or more nucleotides, and more preferably about 20 or more nucleotides. The 3' protruding end may be preferably DNA, more preferably DNA of at least 2 nucleotides in length, and even more preferably DNA of 2-4 nucleotides in length.

An agent capable of causing RNAi useful for the invention may be artificially synthesized (chemically or biochemically) or naturally occurring. There is substantially no difference therebetween in terms of the effect of the present invention. A chemically synthesized agent is preferably purified by liquid chromatography or the like.

An agent capable of causing RNAi used in the present invention can also be produced *in vitro*. In this synthesis system, T7 RNA polymerase, and T7 promoter can be used to synthesize antisense and sense RNAs from template DNA. These RNAs are annealed and thereafter are introduced into a cell.

dsRNA can be delivered to the patient using any means known in the art. Examples of methods of delivering dsRNA to a patient are described in, for example, US 20040180357, US 20040203024 and 20040192629.

### Agents for treating a degenerative disease

When treating a degenerative disease, the agent can be any factor or treatment which selectively or non-selectively results in the destruction, limits the function of, or the inhibition of the production, of regulator cells. For example, a CD4+ specific antibody could be used to specifically target CD4+ T cells. However, in some instances a non-selective agent could be used, such as an anti-proliferative drug, an anti-metabolic drug or radiation, each of which target dividing cells. In particular, as with other cell types, effector cells are particularly vulnerable to destruction by anti-mitotic (anti-proliferative) drugs or spindle poisons (e.g. vinblastine or paclitaxel) when dividing and specifically in mitosis.

Apart from reference to CD8+ specific antibodies, each of the above mentioned agents are also useful for treating a degenerative disease. With regard to dsRNA, the dsRNA molecule can be specific for mRNAs expressed only in regulator cells.

Recent studies have suggested that CD4+CD25+ T cells play an important role in regulating immune cells directed against self antigens (Salomon et al, 2000; Suri-Payer and Cantor, 2001). Furthermore, targeting CD4+CD25+ T cells has been shown to enhance the ability of an animal to control tumour growth (Onizuka et al., 1999; Shimizu et al., 1999; Sutmuller et al., 2001). Accordingly, CD4+CD25+ T cells could be acting as regulator cells as used herein. The activity of CD4+CD25+ T cells can be downregulated by anti-GITR, anti-CD28 and/or anti-CTLA-4 (Read et al., 2000; Takahashi et al., 2000; Shimizu et al., 2002). Thus, these antibodies may be useful as agents for use in the methods of the present invention.

### Monitoring of Patients

In most instances, the time point that the agent is to be administered will need to be empirically determined in subjects at different stages of disease as their immune response kinetics may vary. Other factors such as the general health of the subject and/or the genetic makeup of the subject will also impact upon when is the appropriate time to administer the agent.

Techniques known in the art can be used to monitor the growing population of effector cells during the "cycle".

serial blood samples can be collected and quantitatively screened for T cell subsets (such as CD4+ and/or CD8+) by FACS analysis.

Monitoring may need to be very frequent, for example as often as every few hours, to ensure the correct time point is selected for administration of the agent. Preferably, the monitoring is conducted at least every 48 hours. More preferably, the monitoring is conducted at least every 24 hours.

Optimally, the monitoring is continued to determine the affect of the agent. Insufficient ablation, re-emergence of the effector cells or regulator cells (depending on the disease state being treated) will mean that the method of the present invention should be repeated. Such repeated cycles of treatment may generate immunological memory. It is therefore possible that the present invention, used in repetitive mode, may provide some prophylactic protective effect.

### Vaccines

Vaccines used in the present invention will result in an immune against a protein (antigen) characteristic of a degenerative disease. Such vaccine will comprise at least one antigen, or a polynucleotide encoding said antigen. The vaccine can be provided as any form known in the art such as, but not limited to, a DNA vaccine, ingestion of a transgenic organism expressing the antigen, or composition comprising the antigen.

As used herein, an "antigen" is any polypeptide sequence that contains an epitope which is capable of producing an immune response against the disease.

Antigens which are capable of raising an immune response against a degenerative disease are known in the art. Examples are described in WO 2005/072777 and Gelinas et al. (2004) for raising an immune response to treat Alzheimer's disease, and WO 2005/034995 for raising an immune response to treat prion related diseases.

The antigen can be provided in any manner known in the art which leads to an immune response. An antigen can be, for example, native, recombinant or synthetic.

Vaccines may be prepared from one or more antigens. The preparation of vaccines which contain an antigen is known to one skilled in the art. Typically, such vaccines are prepared as injectables, or orals, either as liquid solutions or suspensions; solid forms suitable for solution in, or suspension in, liquid prior to injection or oral consumption may also be prepared. The preparation may also be emulsified, or the protein encapsulated in liposomes. The antigen is often mixed with carriers/excipients which are pharmaceutically acceptable and compatible with the active ingredient. Suitable carriers/excipients are, for example, water, saline, dextrose, glycerol, ethanol, or the like and combinations thereof.

In addition, if desired, the vaccine may contain minor amounts of auxiliary substances such as wetting or emulsifying agents, pH buffering agents, and/or adjuvants which enhance the effectiveness of the vaccine.

Typically, vaccines comprise an adjuvant. As used herein, the term "adjuvant" means a substance that non-specifically enhances the immune response to an antigen. Examples of adjuvants which may be effective include but are not limited to: N-acetyl-muramyl-L-threonyl-D-isoglutamine (thr-MDP), N-acetyl-nor-muramyl-L-alanyl-D-isoglutamine (CGP 11637, referred to as nor-MDP), N-acetylinuramyl-L-alanyl-D-isoglutaminyl-L-alanine-2-(1'-2'-dipalmitoyl-sn-glycero-3-hydroxyphosphoryloxy)-ethylamine (CGP 19835A, referred to as MTP-PE), and RIBI, which contains three components extracted from bacteria, monophosphoryl lipid A, trehalose dimycolate and cell wall skeleton (MPL+TDM+CWS) in a 2% squalene/Tween 80 emulsion. Further examples of adjuvants include aluminium hydroxide, aluminium phosphate, aluminium potassium sulfate (alum), bacterial endotoxin, lipid X, *Corynebacterium parvum* (*Propionobacterium acnes*), *Bordetella pertussis*, polyribonucleotides, sodium alginate, lanolin, lysolecithin, vitamin A, saponin, liposomes, levamisole, DEAE-dextran, blocked copolymers or other synthetic adjuvants. Such adjuvants are available commercially from various sources, for example, Merck Adjuvant 65 (Merck and Company, Inc., Rahway, N.J.) or Freund's Incomplete Adjuvant and Complete Adjuvant (Difco Laboratories, Detroit, Michigan).

The proportion of antigen and adjuvant can be varied over a broad range so long as both are present in effective amounts. For example, aluminium hydroxide can be present in an amount of about 0.5% of the vaccine mixture (Al₂O₃ basis). Conveniently, the vaccines are formulated to contain a final concentration of antigenic polypeptide in the range of from 0.2 to 200 µg/ml, preferably 5 to 50 µg/ml, most preferably 15 µg/ml.

After formulation, the vaccine may be incorporated into a sterile container which is then sealed and stored at a low temperature, for example 4°C, or it may be freeze-dried. Lyophilisation permits long-term storage in a stabilised form.

The vaccines are conventionally administered parenterally, by injection, for example, either subcutaneously or intramuscularly. Additional formulations which are suitable for other modes of administration include suppositories and, in some cases, oral formulations. For suppositories, traditional binders and carriers may include, for example, polyalkylene glycols or triglycerides; such suppositories may be formed from mixtures containing the active ingredient in the range of 0.5% to 10%, preferably 1% to 2%. Oral formulations include such normally employed excipients as, for example, pharmaceutical grades of mannitol, lactose, starch, magnesium stearate, sodium saccharine, cellulose, magnesium carbonate, and the like. These compositions take the form of solutions, suspensions, tablets, pills, capsules, sustained release formulations or powders and contain 10% to 95% of active ingredient, preferably 25% to 70%. Where the vaccine composition is lyophilised, the lyophilised material may be reconstituted prior to administration, e.g. as a suspension. Reconstitution is preferably effected in buffer.

Capsules, tablets and pills for oral administration to a patient may be provided with an enteric coating comprising, for example, Eudragit "S", Eudragit "L", cellulose acetate, cellulose acetate phthalate or hydroxypropylmethyl cellulose.

DNA vaccination involves the direct *in vivo* introduction of DNA encoding an antigen into tissues of a subject for expression of the antigen by the cells of the subject's tissue. Such vaccines are termed herein "DNA vaccines" or "nucleic acid-based vaccines". DNA vaccines are described in US 5,939,400, US 6,110,898, WO 95/20660 and WO 93/19183, the disclosures of which are hereby incorporated by reference in their entireties.

To date, most DNA vaccines in mammalian systems have relied upon viral promoters derived from cytomegalovirus (CMV). These have had good efficiency in both muscle and skin inoculation in a number of mammalian species. A factor known to affect the immune response elicited by DNA immunization is the method of DNA delivery, for example, parenteral routes can yield low rates of gene transfer and produce considerable variability of gene expression. High-velocity inoculation of plasmids, using a gene-gun, enhanced the immune responses of mice, presumably because of a greater efficiency of DNA transfection and more effective antigens presentation by dendritic cells. Vectors containing the nucleic acid-based vaccine used in the invention may also be introduced into the desired host by other methods known in the art, e.g., transfection, electroporation, microinjection, transduction, cell fusion, DEAE dextran, calcium phosphate precipitation, lipofection (lysosome fusion), or a DNA vector transporter.

Transgenic plants producing a antigenic polypeptide can be constructed using procedures well known in the art. A number of plant-derived edible vaccines are currently being developed for both animal and human pathogens. Immune responses have also resulted from oral immunization with transgenic plants producing virus-like particles (VLPs), or chimeric plant viruses displaying antigenic epitopes. It has been suggested that the particulate form of these VLPs or chimeric viruses may result in greater stability of the antigen in the stomach, effectively increasing the amount of antigen available for uptake in the gut.

### EXAMPLES

### Example 1

Provided below are examples of typical assays used to monitor some acute phase inflammatory markers.

### C-Reactive Protein

C-Reactive Protein was measured using a DADE Behring Dimension RxL Chemistry Analyser, with reagents and calibrators supplied by Dade Behring Diagnostics (Sydney, Australia) (reagent-Cat No. DF-34; calibrators Cat. No. DC-34).

The CRP method is based on a particle enhanced turbidimetric immunoassay technique. Latex particles coated with antibody to C-Reactive Protein aggregate in the presence of C-Reactive Protein in the sample. The increase in turbidity which accompanies aggregation is proportional to the C-Reactive Protein concentration.

| INTRA-ASSAY PRECISION | | | INTER-ASSAY PRECISION | | |
|---|---|---|---|---|---|
| MEAN | CV | N | MEAN | CV | N |
| mg/L | | | mg/L | | |
| 3.4 | 4.3% | 20 | 4.6 | 5.6% | 64 |
| 57.5 | 2.3% | 20 | 37.0 | 3.0% | 64 |
| 225.8 | 2.0% | 20 | | | |

| | | | | | |
|---|---|---|---|---|---|
| REFERENCE RANGE: 0-5 mg/L ANALYTICAL RANGE: 0.5 - 500 mg/L | | | | | |

### Interleukin 2 Receptor (IL2R)

The receptor of the cytokine interleukin 2 (IL2R) is measured by a commercial automated chemiluminescent Enzyme Immuno Assay (EIA) using an Immulite Analyser from Diagnostic Products Corporation (Los Angeles, CA, USA).

This is a competitive immunoassay using Alkaline Phosphatase labelled IL2R as tracer and adamantyl dioxetane as luminescent substrate for ALP enzyme.

All reagents and calibrators are supplied in kit form by DPC - Cat No. LKIPZ.

### Analytical performance:

| | MEAN | SD | CV % |
|---|---|---|---|
| LEVEL 1 | 213 U/mL | 13 | 6.1 |
| LEVEL 2 | 752 U/mL | 49 | 6.5 |
| LEVEL 3 | 2463 U/mL | 189 | 7.7 |

| | | | |
|---|---|---|---|
| ANALYTICAL RANGE: 5-7,500 U/mL REFERENCE RANGE: 223-710 U/mL* *Study performed on 87 apparently healthy adults. | | | |

### Interleukin 6

The cytokine interleukin 6 is measured by a commercial automated chemiluminescent Enzyme Immuno Assay (EIA) using an Immulite Analyser from Diagnostic Products Corporation (Los Angeles, CA, USA).

This is a competitive immunoassay using Alkaline Phosphatase labelled IL-6 as tracer and adamantyl dioxetane as luminescent substrate for ALP enzyme.

All reagents and calibrators are supplied in kit form by DPC - Cat No. LK6PZ.

### Analytical performance:

| | MEAN | SD | CV % |
|---|---|---|---|
| LEVEL 1 | 88 pg/mL | 4.5 | 5.1 |
| LEVEL 2 | 230 pg/mL | 12.2 | 5.3 |
| LEVEL 3 | 638 pg/mL | 46.6 | 7.3 |

| | | | |
|---|---|---|---|
| ANALYTICAL RANGE: 2-1000 pg/mL REFERENCE RANGE: < 4.1 pg/mL* *Study performed on 60 apparently healthy laboratory volunteers. | | | |

### Interleukin 10

The cytokine interleukin 10 is measured by a commercial automated chemiluminescent Enzyme Immuno Assay (EIA) using an Immulite Analyser from Diagnostic Products Corporation, Los Angeles, Ca USA.

This is a competitive immunoassay using Alkaline Phosphatase labelled IL-10

as tracer and adamantyl dioxetane as luminescent substrate for ALP enzyme.

All reagents and calibrators are supplied in kit form by DPC - Cat No. LKXPZ.

### Analytical performance:

| | MEAN | SD | CV % |
|---|---|---|---|
| LEVEL 1 | 18.2 pg/mL | 1.8 | 9.9 |
| LEVEL 2 | 46.0 pg/mL | 2.2 | 4.8 |
| LEVEL 3 | 177 pg/mL | 8.0 | 4.5 |

| | | | |
|---|---|---|---|
| ANALYTICAL RANGE: 5-1000 pg/mL REFERENCE RANGE: < 9.1 pg/mL* *Study performed on 55 apparently healthy adults. | | | |

### Scrum Anayloid A

Polystyrene particles coated with antibodies to human SAA are agglutinated when mixed with samples containing SAA. The intensity of the scattered light in the nephelometer depends on the concentration of the analyte in the sample and consequently its concentration can be determined by comparison with dilutions of a standard of known concentration.

| | | |
|---|---|---|
| IMPRECISION: | CV 4.7% @ 192 mg/L | N=404 |
| | CV 2.8% @ 7.0 mg/L | N=40 |

| | | |
|---|---|---|
| REFERENCE RANGE: In a population with normal serum CRP levels (95^{th} percentile = 5.0 mg/L N=483) the 95^{th} percentile for N Latex SAA was found to be at 6.4 mg/L ANALYTICAL RANGE: 3.0 - 200 mg/L | | |

### Complement C3

The automated method used to measure complement C3 concentration in serum samples by nephelometric analysis using a Dade Behring ProSpect analyzer with reagents and calibrators supplied by Dade Behring Diagnostics (Sydney, Australia).

Soluble antigen solution (sample) and specific antibodies (antiserum Cat No. OSAP15) are mixed in the reaction cuvettes. Insoluble antigen - antibody complexes form immediately, producing turbidity in the mixture and increasing the amount of light scattered by the solution. Following an incubation period the absorbance of the solution is measured at the analytical wavelength.

| | | |
|---|---|---|
| IMPRECISION: | CV 5.5% @ 1.05 g/L | N=61 |
| | CV 3.2% @ 2.70 g/L | N=61 |

| | | |
|---|---|---|
| REFERENCE RANGE: 0.81-1.85 g/L ANALYTICAL RANGE: 0.10 - 3.50 g/L | | |

### Complement C4

The automated method used to measure complement C4 concentration in serum samples by nephelometric analysis using a Dade Behring ProSpect analyzer with reagents and calibrators supplied by Dade Behring Diagnostics (Sydney, Australia).

Soluble antigen solution (sample) and specific antibodies (antiserum Cat No. OSAO 15) are mixed in the reaction cuvettes. Insoluble antigen - antibody complexes form immediately, producing turbidity in the mixture and increasing the amount of light scattered by the solution. Following an incubation period the absorbance of the solution is measured at the analytical wavelength.

| | | |
|---|---|---|
| IMPRECISION: | CV 4.7% @ 0.20 g/L | N=61 |
| | CV 3.8% @ 0.53 g/L | N=61 |

| | | |
|---|---|---|
| REFERENCE RANGE: 0.10 - 0.40 g/L ANALYTICAL RANGE: 0.03 -1.50 g/L | | |

### Example 2

As the skilled address is aware, there are similarities between an autoimmune disease and cancer. More specifically, an autoimmune disease is characterized by an immune response against self antigens. In a similar fashion, the immune system of a cancer patient recognises the overexpression of antigens (cancer antigens) by cancerous cells and raises an immune response against these cells. However, at least in some circumstances, the immune response to the cancer cells does not effectively control the cancer resulting in the persistence of the disease. Thus, immune system cycling in cancer patients is a suitable model to show similar cycling in subjects with an autoimmune disease.

The patient was a 71 year old female designated herein "Mrs FO". Previously Mrs FO was diagnosed with ovarian cancer, received surgery and several rounds of standard chemotherapy. Patient represented with elevated CA125 at 200U/ml prior to monitoring.

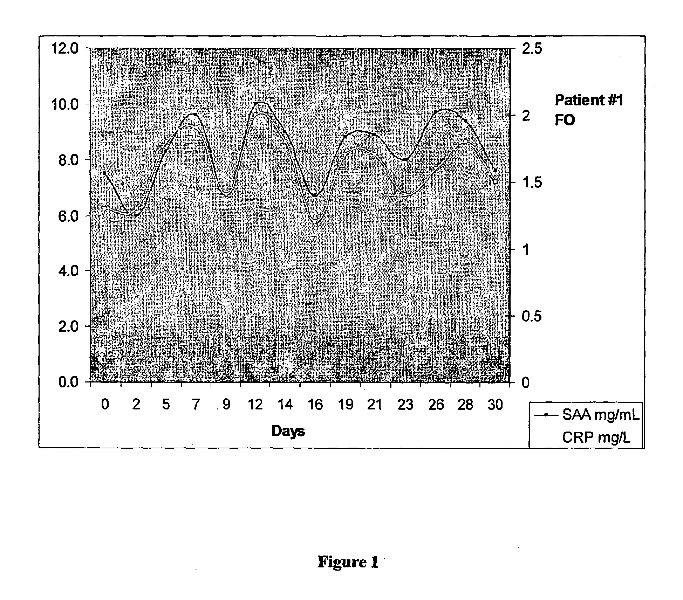

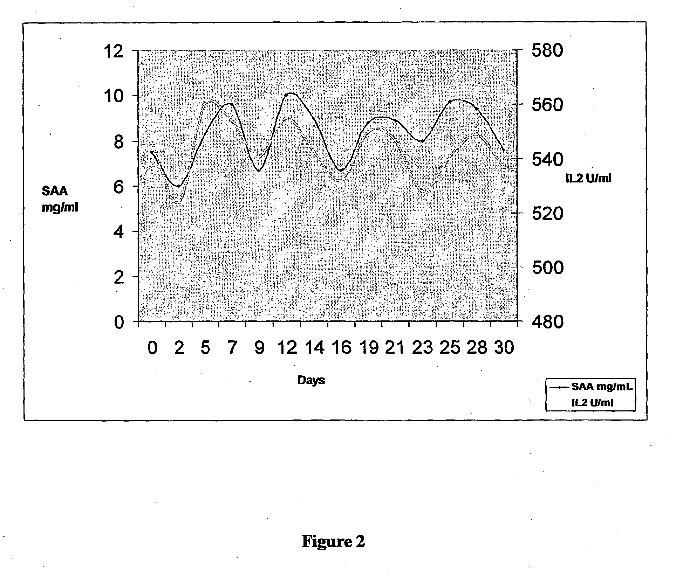

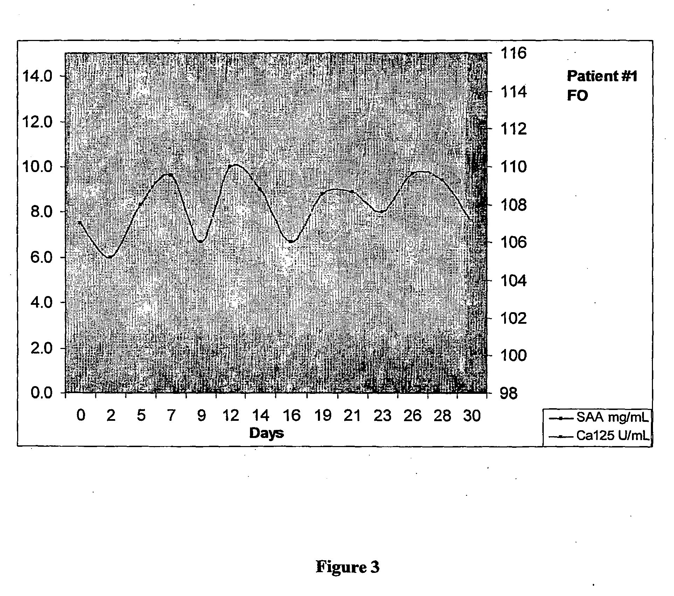

Patient was monitored (bled) every Monday, Wednesday & Friday for 4 weeks. A well described near synchronous and regular oscillation with a 7 /14 day periodicity showing a close correlation between CRP, SAA & IL-2 serum measurements (see Figures 1 and 2). More interestingly, Figure 3 which shows CRP & CA125 versus time, the CRP and CA125 oscillations are out of phase, indicating an inverse relationship between the immune system and the cancer marker.

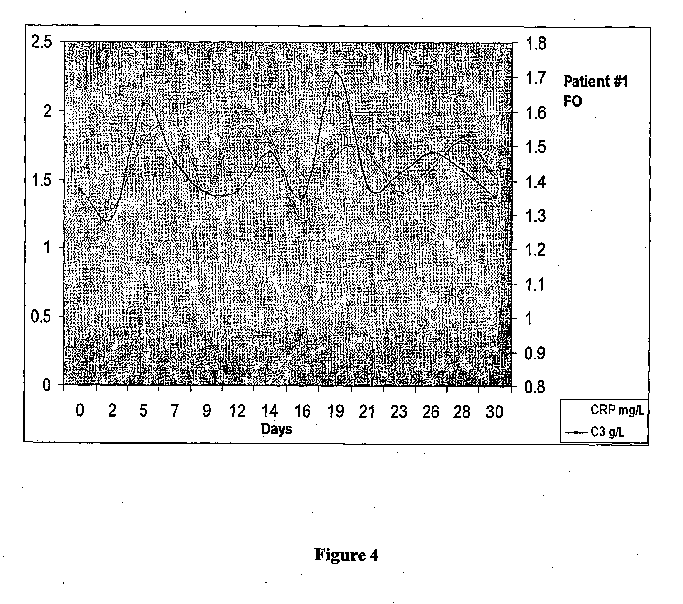

Figure 4 shows the relationship over time between SAA and complement factor C3. Note that the two major C3 peaks are approximately 14 days apart and coincide with alternating SAA peaks which are also approximately 14 days apart.

Further examples of the immune system cycling in cancer patients is described in WO 2005/040816.

### Example 3

A search is preformed for a suitable donor for a patient with chronic glomerulonephritis requiring a renal cadaveric allograft. The allograft is performed using standard surgical procedures. Upon completion of the allograft the patient is monitored for serum amyloid A (SAA) levels. When SAA levels begin to increase this indicates that an immune response is being mounted against the graft characterized by the production of effector cells against the graft (also referred, to in the art as a rejection).

An example of SAA and c-reactive (CRP) protein levels following a renal cadaveric renal allograft is described by Maury and Teppo (1984). As can be seen in Figure 4 of Maury and Teppo (1984), there is an approximately 15 day period in between peak levels of CRP and SAA linked to rejection episodes following transplant indicating that effector cells are cycling in the patient studied.

Vinblastine is administered at a standard dose such as 3-4 mg/m² intravenously (Casciato and Lowitz, 1995) as SAA levels begin to rise. In this instance, vinblastine will target dividing cells, such as the effector cells, alleviating the rejection episode.

### Example 4

A patient with juvenile chronic arthritis is monitored for SAA levels. Upon establishment of a clear cycling in SAA levels vincristine is administered, using a standard dosage, as SAA levels begin to rise. In this instance, vincristine will target dividing cells, such as the effector cells at this point in the immune cycle, alleviating symptoms of the disease.

An example of SAA protein levels cycling in a juvenile chronic arthritis patient is described by Elliott et al. (1997). As can be seen from Figure 2 of Elliott et al. (1997), there is an approximately 14 day period in between peak levels of SAA. If monitoring was performed more frequently it is believed similar cycling would be more clear for the CRP levels as well.

### Example 5

A patient with Alzheimer's disease is monitored for c-reactive protein levels. Upon establishment of a clear cycling in c-reactive protein levels vincristine is administered, using a standard dosage, as c-reactive protein have peaked. In this instance, vincristine will target dividing cells, such as the regulator cells at this point in the immune cycle, alleviating symptoms of the disease.

### Cross-Reference to Related Applications

The present application claims priority from Provisional Patent Application No. 2004905118 filed on 8 September 2004.

### REFERENCES

Annunziato, F. et al. (2002) J. Exp. Med. 193:1285-1294.
Babbe, H. et al. (2000) J. Exp. Med. 192:393-404.
Bottazzo, G.F. et al. (1985) New Engl. J. Med. 313:353-360.
Bretz, J.D. et al. (1999) J. Biol. Chem. 274:25433-25438.
Casciato, D.A. and Lowitz, B.B. (1995) Manual of Clinical Oncology. Third Edition.
Little Brown and Company, Boston.
Eda, S., et al. (1998) J. Clin. Lab. Analysis 12:137-144.
Elliot, M. J. et al. (1997) Br. J. Rheumatol. 36:589-593.
Fu, S. et al. (2004) Am. J. Transplant. 4:65-78.
Gelinas, D.S. et al. (2004) Proc. Natl. Acad. Sci, USA 101:14657-14662.
Goverman, J. (1999) Immunol. Rev. 169:147-159.
Hill, G.R. et al. (1997) Blood 90:3204-3213.
Jonuleit, H. et al. (2001) J. Exp. Med. 193:1285-1294.
Kimura, M., et al. (2001) Cancer 92:2072-2075.
Kohm, A.P. et al. (2002) J. Immunol. 169:4712-4716.
Liuzzo, G., et al. (1994) New Engl. J. Med. 331:417-424:
Maury, C.P.J. and Teppo, A.M. (1984) Clin. Nephrol. 22:284-292.
Monsonego, A. and Weiner, H.L. (2003) Science 302:834-838.
Morgan, M.E. et al. (2003) Arthritis Rheum. 48:1452-1460.
Murphy, W.J. and Blazar, B.R (1999) Curr. Opin. Immunol. (1999) 11:509-515.
O'Hanlon, D.M., et al. (2002) Anticancer Res. 22:1289-1294.
O'Hara, R., et al. (2000) Arthritis Res. 2:142-144.
Onizuka, S., et al. (1999) Cancer Res. 59:3128-3133.
Price, C.P., et al. (1987) J. Immunol. Methods 99:205-211.
Read, S, et al. (2000) J. Exp. Med. 192:295-302.
Salomon, B. et al. (2000) Immunity 12:431-440.
Santamaria, P. (2001) Curr. Opin. Immunol. 13:663-669.
Senju, O., et al. (1983) Jap. J. Clin. Lab. Automation 8:161-165.
Shimizu, J., et al. (1999) J. Immunol. 163:5211-5218.
Shimizu, J., et al. (2002) Nature Immunol. 3:135-142.
Speiser, D.E. et al. (1997) J. Immunol. 158:5185-5190.
Suri-Payer, E. and Cantor, H. (2001) J. Autoimmunity 16:115-123.
Sutmuller, R. P., et al.(2001) J. Exp. Med. 194:823-832.
Takahashi, T., et al. (2000) J. Exp. Med. 192:303-310.
Verma, S. (2000) Eur. J. Immunol. 30:1191-1202.
von Herrath, M.G. and Harrison, L.C. (2003) Nature Rev. 3:223-232.
Weinstein, P.S. et al. (1984) Scand. J. Immunol. 19:193-198.
Wong, F. et al. (1999) Nat. Med. 9:1026-1031..
Wu, A.J. et al. (2002) Proc. Natl. Acad. Sci, USA 99:12287-12292.

## Claims

1. A method for analysing immune system cycling to determine when an agent should be administered to a patient suffering from a disease **characterized by** the production of effector T cells or a degenerative disease, the method comprising monitoring the patient, or samples obtained therefrom, for fluctuations in at least one of: a) effector T cell numbers and/or activity, b) regulator T cell numbers and/or activity, c) a marker molecule associated with the disease, and/or d) an immune system marker;
wherein if the disease is **characterized by** the production of effector T cells:
(i) the disease is an autoimmune disease or transplantation rejection;
(ii) the agent inhibits the production, limits the function of and/or activity of effector T cells and is selected from the group consisting of anti-proliferative drugs, anti-metabolic drugs, radiation, dsRNA and antibodies; and
(iii) the timing of when the agent should be administered is when, or just before, effector T cells begin clonally expanding such that effector T cell expansion does not occur, and/or effector T cell numbers are reduced or abolished;
and wherein if the disease is a degenerative disease:
(i) the disease is a condition that results in the loss of cells, and is Alzheimer's disease or a prion related disease; and
(ii) the agent:
(a) inhibits the production, limits the function of and/or activity of regulator T cells and is selected from the group consisting of anti-proliferative drugs, anti-metabolic drugs, radiation, dsRNA, and antibodies; and the timing of when the agent should be administered is such that the agent exerts a proportionally greater effect against the regulator T cells than the effector T cells; or
(b) is a vaccine; and the timing of when the vaccine should be administered is such that the vaccine boosts the innate immune response, producing increased numbers and/or activity of effector T cells, before the emergence of regulator T cells.

2. The method of claim 1, wherein the immune system marker is an acute phase inflammatory marker.

3. The method of claim 1 or claim 2, wherein the patient is suffering from a disease **characterized by** the production of effector T cells, and wherein the agent is administered between when the levels of an acute phase inflammatory marker have reached their lowest point and before the marker peaks in the next cycle.

4. The method according to any one of claims 1 to 3, wherein the effector T cells are CD8+CD4- T cells.

5. The method according to any one of claims 1 to 4, wherein the regulator T cells are CD4+CD8- T cells.

6. The method according to any one of claims 1 to 5, wherein the patient is monitored for a period of at least 7 days.

7. The method according to any one of claims 1 to 6, the patient is monitored at least about every 3 days.

8. Use of an agent which inhibits the production, limits the function of and/or activity of effector T cells in the manufacture of a medicament for the treatment of a disease **characterized by** the production of effector T cells, the treatment comprising:
i) analysing immune system cycling by monitoring a patient suffering from the disease for fluctuations in at least one of:
a) number and/or activity of regulator T cells,
b) number and/or activity of effector T cells,
c) a marker molecule associated with the disease, and/or
d) an immune system marker, and
ii) exposing the patient to an agent to treat the disease,
wherein:
(a) the disease is an autoimmune disease or transplantation rejection;
(b) the agent is selected from the group consisting of anti-proliferative drugs, anti-metabolic drugs, radiation, dsRNA and antibodies; and
(c) the agent is administered when, or just before, effector T cells begin clonally expanding such that effector T cell expansion does not occur, and/or effector T cell numbers are reduced or abolished.

9. Use of an agent in the manufacture of a medicament for the treatment of a degenerative disease, the treatment comprising:
i) analysing immune system cycling by monitoring a patient suffering from the disease for fluctuations in at least one of:
a) number and/or activity of regulator T cells,
b) number and/or activity of effector T cells,
c) a marker molecule associated with the disease, and/or
d) an immune system marker, and
ii) exposing the patient to an agent to treat the disease,
wherein:
(a) the disease is a condition that results in the loss of cells and is Alzheimer's disease or a prion related disease; and
(b) the agent:
(i) inhibits the production, limits the function of and/or activity of regulator T cells and is selected from the group consisting of anti-proliferative drugs, anti-metabolic drugs, radiation, dsRNA and antibodies; and the timing of administration of the agent is such that the agent exerts a proportionally greater effect against the regulator T cells than the effector T cells; or
(ii) is a vaccine; and the timing of administration of the vaccine is such that the vaccine boosts the innate immune response, producing increased numbers and/or activity of effector T cells, before the emergence of regulator T cells.

## Patentansprüche

1. Verfahren zur Analyse zyklischer Schwankungen des Immunsystems zum Bestimmen, wann ein Mittel einem Patienten, der an einer Krankheit, die durch die Herstellung von Effektor-T-Zellen gekennzeichnet ist, oder einer degenerativen Krankheit leidet, verabreicht werden sollte, wobei das Verfahren das Überwachen des Patientens oder davon erhaltener Proben in Bezug auf Schwankungen bei wenigstens einem der folgenden umfasst: a) Anzahl und/oder Aktivität der Effektor-T-Zellen, b) Anzahl und/oder Aktivität regulatorischer T-Zellen, c) einem Markermolekül, das mit der Erkrankung in Verbindung gebracht wird und/oder d) einem Immunsystemmarker,
wobei, wenn die Krankheit durch die Herstellung von Effektor-T-Zellen charakterisiert ist:
(i) die Erkrankung eine Autoimmunerkrankung oder Transplantatabstoßung ist,
(ii) das Mittel die Herstellung von Effektor-T-Zellen hemmt, deren Funktion und/oder Aktivität begrenzt und ausgewählt ist aus der Gruppe, bestehend aus antiproliferativen Medikamenten, antimetabolischen Medikamenten, Strahlung, dsRNA und Antikörpern, und
(iii) die Terminierung, wann das Mittel verabreicht werden sollte dann ist, wenn Effektor-T-Zellen beginnen sich klonal zu vermehren oder kurz davor, so dass eine Vermehrung von Effektor-T-Zellen nicht auftritt und/oder Effektor-T-Zellanzahlen reduziert oder vernichtet werden,
und wenn die Erkrankung eine degenerative Erkrankung ist:
(i) die Erkrankung ein Zustand ist, der zum Verlust von Zellen führt, und Alzheimer oder eine prionenbedingte Erkrankung ist und
(ii) das Mittel:
(a) die Herstellung von regulatorischen T-Zellen hemmt, deren Funktion und/oder Aktivität begrenzt und ausgewählt ist aus der Gruppe, bestehend aus antiproliferativen Medikamenten, antimetabolischen Medikamenten, Strahlung, dsRNA und Antikörpern, und die Terminierung, wann das Mittel verabreicht werden sollte so ist, dass das Mittel einen proportional größeren Effekt gegen regulatorische T-Zellen ausübt als die Effektor-T-Zellen, oder
(b) ein Impfstoff ist und die Terminierung, wann der Impfstoff verabreicht werden sollte so ist, dass der Impfstoff die immanente Immunantwort verstärkt, wobei größere Anzahlen von Effektor-T-Zellen hergestellt werden und/oder deren Aktivität verstärkt wird, bevor regulatorische T-Zellen auftreten.

2. Verfahren nach Anspruch 1, wobei der lmmunsystemmarker ein Entzündungsmarker der akuten Phase ist.

3. Verfahren nach Anspruch 1 oder Anspruch 2, wobei der Patient an einer Erkrankung leidet, die durch die Herstellung von Effektor-T-Zellen gekennzeichnet ist und wobei das Mittel zwischen dem Zeitpunkt verabreicht wird, wenn die Mengen an inflammatorischem Marker der akuten Phase ihren geringsten Punkt erreicht haben und bevor der Marker in dem nächsten Zyklus seinen Höchstwert erreicht.

4. Verfahren gemäß einem der Ansprüche 1 bis 3, wobei die Effektor-T-Zellen CD8+CD4- T-Zellen sind.

5. Verfahren gemäß einem der Ansprüche 1 bis 4, wobei die regulatorischen T-Zellen CD4+CD8- T-Zellen sind.

6. Verfahren gemäß einem der Ansprüche 1 bis 5, wobei der Patient für einen Zeitraum von wenigstens 7 Tagen überwacht wird.

7. Verfahren gemäß einem der Ansprüche 1 bis 6, wobei der Patient wenigstens ungefähr jeden dritten Tag überwacht wird.

8. Verwendung eines Mittels, das die Herstellung von Effektor-T-Zellen hemmt, deren Funktion und/oder Aktivität begrenzt, bei der Herstellung eines Medikaments zur Behandlung einer Erkrankung, die durch die Herstellung von Effektor-T-Zellen gekennzeichnet ist, wobei die Behandlung umfasst, dass man:
(i) zyklische Schwankungen des Immunsystems durch Überwachen eines Patienten, der an einer Erkrankung leidet, auf Schwankungen bei wenigstens einem der folgenden analysiert:
a) Anzahl und/oder Aktivität von regulatorischen T-Zellen,
b) Anzahl und/oder Aktivität von Effektor-T-Zellen,
c) einem IVlarkermolekül, das mit der Erkrankung assoziiert ist, und/oder
d) einem lmmunsystemmarker, und
(ii) den Patienten einem Mittel zur Behandlung der Erkrankung aussetzt,
wobei:
(a) die Erkrankung eine Autoimmunerkrankung oder Transplantatabstoßung ist,
(b) das Mittel ausgewählt ist aus der Gruppe, bestehend aus antiproliferativen Medikamenten, antimetabolischen Medikamenten, Strahlung, dsRNA und Antikörpern, und
(c) das Mittel verabreicht wird, wenn die Effektor-T-Zellen beginnen sich klonal zu vermehren oder kurz davor, so dass eine Vermehrung von Effektor-T-Zellen nicht auftritt und/oder Effektor-T-Zellenanzahlen reduziert oder vernichtet werden.

9. Verwendung eines Mittels zur Herstellung eines Medikaments für die Behandlung einer degenerativen Erkrankung, wobei die Behandlung umfasst, dass man:
(i) zyklische Schwankungen des Immunsystems durch Überwachen eines Patienten, der an einer Erkrankung leidet, auf Schwankungen bei wenigstens einem der folgenden analysiert:
a) Anzahl und/oder Aktivität von regulatorischen T-Zellen,
b) Anzahl und/oder Aktivität von Effektor-T-Zellen,
c) einem Markermolekül, das mit der Erkrankung assoziiert ist, und/oder
d) einem lmmunsystemmarker, und
(ii) den Patienten einem Mittel zur Behandlung der Erkrankung aussetzt,
wobei:
(a) die Erkrankung ein Zustand ist, der zum Verlust von Zellen führt, und Alzheimer oder eine prionenbedingte Erkrankung ist und
(b) das Mittel:
(i) die Herstellung von regulatorischen T-Zellen hemmt, deren Funktion und/oder Aktivität begrenzt und ausgewählt ist aus der Gruppe, bestehend aus antiproliferativen Medikamenten, antimetabolischen Medikamenten, Strahlung, dsRNA und Antikörpern, und die Terminierung, wann das Mittel verabreicht werden sollte so ist, dass das Mittel einen proportional größeren Effekt gegen regulatorische T-Zellen ausübt als die Effektor-T-Zellen, oder
(ii) ein Impfstoff ist und die Terminierung, wann der Impfstoff verabreicht werden sollte so ist, dass der Impfstoff die immanente Immunantwort verstärkt, wobei größere Anzahlen von Effektor-T-Zellen hergestellt werden und/oder deren Aktivität verstärkt wird, bevor regulatorische T-Zellen auftreten.

## Revendications

1. Procédé d'analyse des cycles du système immunitaire pour déterminer quand il faudrait administrer un agent à un patient souffrant d'une maladie **caractérisée par** la production de lymphocytes T effecteurs ou d'une maladie dégénérative, le procédé comprenant le suivi chez le patient, ou dans des échantillons prélevés chez ce patient, des fluctuations d'au moins l'un des éléments suivants: a) le nombre et/ou l'activité des lymphocytes T effecteurs, b) le nombre et/ou l'activité de lymphocytes T régulateurs, c) une molécule de marqueur associée à la maladie, et/ou d) un marqueur du système immunitaire,
où, si la maladie est **caractérisée par** la production de lymphocytes T effecteurs:
(i) la maladie est une maladie auto-immune ou le rejet d'une greffe;
(ii) l'agent inhibe la production, limite la fonction et/ou l'activité des lymphocytes T effecteurs et est choisi dans le groupe constitué par des médicaments antiprolifératifs, des médicaments antimétaboliques, des rayonnements, des ARNds et des anticorps; et
(iii) le moment où devrait être administré l'agent est le moment du début de l'expansion clonale des lymphocytes T effecteurs, ou juste avant, de façon que l'expansion des lymphocytes T effecteurs ne se produise pas, et/ou que le nombre de lymphocytes T effecteurs soit réduit ou supprimé;
et où, si la maladie est une maladie dégénérative,
(i) la maladie est un état qui résulte de la perte de cellules, et est la maladie d'Alzheimer ou une maladie associée à un prion; et
(ii) l'agent:
(a) inhibe la production, limite la fonction et/ou l'activité des lymphocytes T régulateurs et est choisi dans le groupe constitué par des médicaments antiprolifératifs, des médicaments antimétaboliques, des rayonnements, des ARNds, et des anticorps; et le moment où il faudrait administrer l'agent est tel que l'agent exerce un effet proportionnellement plus grand contre les lymphocytes T régulateurs que contre les lymphocytes T effecteurs; ou
(b) est un vaccin; et le moment où il faudrait administrer le vaccin est tel que le vaccin renforce la réponse immunitaire innée, produisant un nombre et/ou une activité accrus de lymphocytes T effecteurs, avant l'émergence de lymphocytes T régulateurs.

2. Procédé selon la revendication 1, dans lequel le marqueur du système immunitaire est un marqueur inflammatoire de phase aiguë.

3. Procédé selon la revendication 1 ou la revendication 2, dans lequel le patient souffre d'une maladie **caractérisée par** la production de lymphocytes T effecteurs, et dans lequel l'agent est administré entre le moment où les niveaux d'un marqueur inflammatoire de phase aiguë ont atteint leur niveau le plus bas et le moment où le marqueur atteint un maximum dans le cycle suivant.

4. Procédé selon l'une quelconque des revendications 1 à 3, dans lequel les lymphocytes T effecteurs sont des lymphocytes T CD8+CD4-.

5. Procédé selon l'une quelconque des revendications 1 à 4, dans lequel les lymphocytes T régulateurs sont des lymphocytes T CD4+CD8-.

6. Procédé selon l'une quelconque des revendications 1 à 5, dans lequel le patient est suivi pendant une période d'au moins 7 jours.

7. Procédé selon l'une quelconque des revendications 1 à 6, dans lequel le patient est suivi au moins environ tous les 3 jours.

8. Utilisation d'un agent qui inhibe la production, limite la fonction et/ou l'activité de lymphocytes T effecteurs, dans la fabrication d'un médicament destiné au traitement d'une maladie **caractérisée par** la production de lymphocytes T effecteurs, le traitement comprenant:
i) l'analyse des cycles du système immunitaire par le suivi chez un patient souffrant de la maladie des fluctuations d'au moins l'un des éléments suivants:
a) le nombre et/ou l'activité des lymphocytes T régulateurs,
b) le nombre et/ou l'activité des lymphocytes T effecteurs,
c) une molécule de marqueur associée à la maladie, et/ou
d) un marqueur du système immunitaire, et
ii) l'exposition du patient à un agent pour traiter la maladie,
où:
(a) la maladie est une maladie auto-immune ou un rejet de greffe;
(b) l'agent est choisi dans le groupe constitué par des médicaments antiprolifératifs, des médicaments antimétaboliques, des rayonnements, des ARNds et des anticorps; et
(c) l'agent est administré au moment du début de l'expansion clonale des lymphocytes T effecteurs, ou juste avant, de façon que l'expansion des lymphocytes T ne se produise pas, et/ou que le nombre de lymphocytes T effecteurs soit réduit ou supprimé.

9. Utilisation d'un agent dans la fabrication d'un médicament destiné au traitement d'une maladie dégénérative, le traitement comprenant:
i) l'analyse des cycles du système immunitaire par le suivi chez un patient souffrant de la maladie des fluctuations d'au moins l'un des éléments suivants:
a) le nombre et/ou l'activité des lymphocytes T régulateurs,
b) le nombre et/ou l'activité des lymphocytes T effecteurs,
c) une molécule de marqueur associée à la maladie, et/ou
d) un marqueur du système immunitaire, et
ii) l'exposition du patient à un agent pour traiter la maladie,
où:
(a) la maladie est un état qui résulte de la perte de cellules, et est la maladie d'Alzheimer ou une maladie associée à un prion; et
(b) l'agent:
(i) inhibe la production, limite la fonction et/ou l'activité des lymphocytes T régulateurs et est choisi dans le groupe constitué par des médicaments antiprolifératifs, des médicaments antimétaboliques, des rayonnements, des ARNds, et des anticorps; et le moment de l'administration de l'agent est tel que l'agent exerce un effet proportionnellement plus grand contre les lymphocytes T régulateurs que contre les lymphocytes T effecteurs; ou
(ii) est un vaccin; et le moment de l'administration du vaccin est tel que le vaccin renforce la réponse immunitaire innée, produisant un nombre et/ou une activité accrus des lymphocytes T effecteurs, avant l'émergence de lymphocytes T régulateurs.
